# EUROPÄISCHE PATENTSCHRIFT

(11) **EP 0 696 580 B1**
(45) Veröffentlichungstag und Bekanntmachung des Hinweises auf die Patenterteilung: **14.01.1998**
(21) Anmeldenummer: 95111812.4
(22) Anmeldetag: 27.07.1995
(51) Int. Cl.: C07C 271/20, C08G 18/18

(54) **Carbonat- und Urethangruppen aufweisende tertiäre Amine und ihre Verwendung als Katalysatoren bei der Herstellung von Polyurethankunststoffen**
Carbonate and urethane groups containing tertiary amines and their use as catalyst in the preparation of polyurethanes
Amines tertiaires ayant des groupes d'uréthane et carbonate et leur application comme catalyseurs dans la préparation des polyuréthanes

(30) Priorität: 09.08.1994 DE 4428108
(43) Veröffentlichungstag der Anmeldung: 14.02.1996
(73) Patentinhaber: BAYER AG, 51368 Leverkusen (DE)
(72) Erfinder: König, Klaus, Dr., D-51519 Odenthal (DE); Liman, Ulrich, Dr., D-40764 Langenfeld (DE); Sanders, Josef, Dr., D-51373 Leverkusen (DE)

(56) Entgegenhaltungen:
- DE-A- 4 030 515

## Beschreibung

Die Erfindung betrifft ein Verfahren zur Herstellung von neuen, Carbonat- und Urethangruppen, sowie gegebenenfalls Hydroxylgruppen aufweisenden tertiären Aminen, die nach diesem Verfahren erhältlichen Verbindungen und ihre Verwendung als Katalysatoren bei der Herstellung von Polyurethankunststoffen, insbesondere bei der Herstellung von Verbundkörpern durch Hinterschäumen von Kunststoffolien mit einem zu Polyurethanschaumstoff ausreagierenden Reaktionsgemisch.

Die DE-OS 40 30 515 beschreibt die Herstellung von tert. Aminogruppen aufweisenden Urethanen durch Umsetzung von cyclischen Carbonaten wie z.B. Ethylen- oder Propylen-carbonat und sowohl primäre als auch tertiäre Aminogruppen aufweisenden Aminen, sowie ihre Verwendung als Katalysatoren zur Herstellung von Polyurethanen. Die resultierenden Urethane sind katalytisch aktiv und besitzen zum Einbau in die Polyurethanmatrix befähigte Hydroxylgruppen. Wie inzwischen anwendungstechnische Untersuchungen gezeigt haben, ist ihr vollständiger Einbau jedoch nicht immer gewährleistet bzw. zu Anfang der Polyaddition noch nicht erfolgt. So können diese Stoffe, ebenso wie andere einbaufähige Katalysatoren wie z.B. alkoxylierte Amine, aufgrund ihrer niedrigen Molmasse in die Umgebung gelangen und so zum sogenannten "Fogging" beitragen. Außerdem besteht z.B. bei der Herstellung von Verbundkörpern durch Hinterschäumen von Kunststoffolien die Gefahr, daß diese Katalysatoren in die Deckmaterialien diffundieren und diese, gegebenenfalls unter dem Einfluß von Sonnenlicht und Wärme, schädigen.

Wie jetzt überraschend gefunden wurde, können die angesprochenen Probleme durch Verwendung der neuen, nachstehend näher beschriebenen erfindungsgemäßen Verfahrensprodukte beseitigt werden. Diese weisen, sehr wahrscheinlich wegen ihres höheren Molekulargewichts, eine weit geringere Tendenz auf, aus den Schaumstoffen zu diffundieren.

Gegenstand der Erfindung ist ein Verfahren zur Herstellung von Carbonat- und Urethangruppen, sowie gegebenenfalls Hydroxylgruppen aufweisenden tert.-Aminen, dadurch gekennzeichnet, daß man
a) eine Polyolkomponente, bestehend aus mindestens einem mehrwertigen Alkohol der Formel

   Q(OH)ₙ (I)

   und
b) eine Aminkomponente, bestehend aus mindestens einem primär-tertiären Diamin der Formel 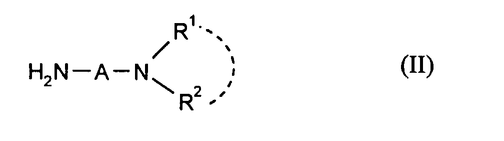 mit
c) Kohlesäurederivaten, ausgewählt aus der Gruppe bestehend aus Carbonaten der Formel

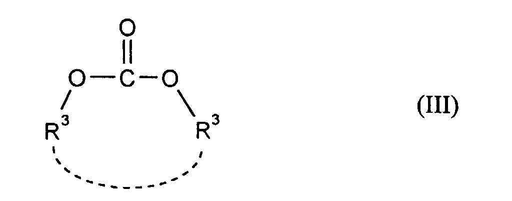

Harnstoff, Phosgen und Bischlorkohlensäureester von Polyolen der Formel (I),
ein- oder zweistufig unter Carbonat- und Urethanbildung zur Reaktion bringt,
mit der Maßgabe, daß das Mengenverhältnis der Komponenten a) und b) einem Molverhältnis von Hydroxylgruppen zu primären Aminogruppen von 4:1 bis 16:1 entspricht und die Kohlensäurederivate c) in solchen Mengen zum Einsatz gelangen, daß auf jedes Mol an Aminen b) 0,5 Mol an Kohlensäurederivat c) und zusätzlich auf jedes Mol an Hydroxylgruppen der Komponente a) 0,2 bis 0,5 weitere Mole an Kohlensäurederivaten c) entfallen, wobei
- Q: für den Rest steht, wie er durch Entfernung von n-Hydroxylgruppen aus einem n-wertigen, gegebenenfalls Ethergruppen aufweisenden aliphatischen Alkohol des Molekulargewichtsbereichs 90 bis 400 erhalten wird,
- n: für 2 oder 3 steht
- A: für einen zweiwertigen aliphatischen Kohlenwasserstoffrest mit 2 bis 6 Kohlenstoffatomen steht, wobei zwischen den Stickstoffatomen mindestens 2 Kohlenstoffatome angeordnet sind,
- R¹ und R²: für gleiche oder verschiedene Reste stehen und Alkylreste mit 1 bis 4 Kohlenstoffatomen bedeuten, die auch zusammen mit dem Stickstoffatom und gegebenenfalls weiteren Heteroatomen einen gesättigten, heterocyclischen, gegenüber den Kohlesäurederivaten c) inerten Ring mit 5 oder 6 Ringgliedern bilden können, und
- R³: jeweils für Phenylreste oder Alkylreste mit 1 bis 4 Kohlenstoffatomen stehen oder zusammen einen Alkylenrest mit 2 oder 3 Kohlenstoffatomen bilden.

Gegenstand der Erfindung sind auch die nach diesem Verfahren erhältlichen Carbonat- und Urethangruppen, sowie gegebenenfalls Hydroxylgruppen aufweisenden tertiären Amine, sowie ihre Verwendung als Katalysatoren bei der Herstellung von Polyurethankunststoffen nach dem Isocyanat-Polyadditionsverfahren, insbesondere bei der Herstellung von Verbundkörpern durch Hinterschäumen von Kunststoffolien mit einem zu Polyurethanschaumstoff ausreagierenden Reaktionsgemisch.

Die erfindungsgemäßen Verfahrensprodukte weisen im allgemeinen einen Gehalt an Carbonatgruppen (berechnet als CO₃, Molekulargewicht = 60) von 5 bis 30 Gew.-%, einen Gehalt an Urethangruppen (berechnet als CHNO₂, Molekulargewicht = 59) von 5 bis 20 Gew.-%, einen Gehalt an alkoholischen Hydroxylgruppen von 0 bis 10 Gew.-% und einen Gehalt an tertiären Aminogruppen (berechnet als N, Atomgewicht = 14) von 1 bis 5 Gew.-% auf.

Ausgangsmaterialien für das erfindungsgemäße Verfahren sind a) zwei- oder dreiwertige Alkohole der nachstehend näher beschriebenen Art, b) primär-tertiäre Diamine der nachstehend näher beschriebenen Art und c) Kohlensäurederivate der nachstehend näher beschriebenen Art.

Die Alkoholkomponente a) besteht aus mindestens einem Alkohol der vorstehend genannten allgemeinen Formel (I). Als Alkoholkomponente a) gut geeignet sind beispielsweise (i) gegebenenfalls Ethergruppen aufweisende aliphatische Diole des Molekulargewichtsbereichs 90 bis 400, vorzugsweise 106 bis 250, (ii) gegebenenfalls Ethergruppen aufweisende aliphatische Triole des Molekulargewichtsbereichs 120 bis 400, vorzugsweise 134 bis 400 und (iii) beliebige Gemische von zwei-und dreiwertigen Alkoholen (i) und (ii).

Beispiele für geeignete zweiwertige Alkohole (i) sind 1,4-Butandiol, 1.5-Pentandiol, 3-Methyl-pentandiol-1.5, 2,2,4-Trimethylhexandiol-1.6, die isomeren Heptandiole, Octandiole, Nonandiole oder Decandiole, sowie die an sich bekannten Anlagerungsprodukte des genannten Molekulargewichtsbereichs von Ethylenoxid und/oder Propylenoxid an derartige Alkohole oder an Ethylenglykol oder Propylenglykol. Bevorzugt sind Di-, Tri- und Tetraethylenglykol sowie Di-, Tri- und Tetrapropylenglykol bzw. deren Mischungen, wobei über den Ethylen-bzw. Propylenoxidanteil die Hydrophilie bzw. die Löslichkeit der Endprodukte in Wasser oder Polyetherpolyol beeinflußt werden kann.

Geeignete dreiwertige Alkohole (ii) sind beispielsweise Trimethylolethan, Trimethylolpropan, sowie die an sich bekannten Anlagerungsprodukte des genannten Molekulargewichtsbereichs von Ethylenoxid und/oder Propylenoxid an derartige Triole, wobei auch hier über den Ethylen- bzw. Propylenoxidanteil die Hydrophilie bzw. Löslichkeit der Endprodukte in Wasser oder Polyetherpolyol beeinflußt werden kann. Bevorzugter mehrwertiger Alkohol (ii) ist Trimethylolpropan.

Bei den primär-tertiären Aminen b) handelt es sich um solche der bereits vorstehend genannten allgemeinen Formel (II), wobei insbesondere solche bevorzugt sind, für welche A für einen Alkylenrest mit 2 bis 4 Kohlenstoffatomen steht und R¹ und R² jeweils für Methylgruppen stehen.

Die Herstellung der primär-tertiären Diamine kann beispielsweise durch Alkylierung mono-geschützter diprimärer Amine, z.B. Aminoalkyl-phthalimide, und anschließende Hydrolyse bzw. Hydrazinolyse oder, vorzugsweise, durch Addition von Aziridin oder Acrylnitril an sekundäre Amine wie z.B. Dimethylamin, Diethylamin, Dipropylamin, Piperidin oder Morpholin und anschließende Hydrierung der cyanethylierten Amine hergestellt werden. Beispiele geeignete Amine b) sind: 1-(Dimethylamino)-3-aminopropan, 1-(Diethylamino)-3-aminopropan, 1-(Di-n-propylamino-3-aminopropan, 1-(Dimethylamino)-2-methyl-3-aminopropan, 1-(Dimethylamino)-4-aminobutan, 1-(Dimethylamino)-5-aminopentan, N-(2-Aminoethyl)-morpholin, N-(3-Aminopropyl)-morpholin, N-(2-Aminoethyl)-piperidin oder N-(3-Aminopropyl)-piperidin. Es ist auch möglich, Mischungen der aufgeführten Amine zu verwenden. Bevorzugt wird 1-(Dimethylamino)-3-aminopropan.

Bei den Kohlensäurederivaten c) handelt es sich um Verbindungen, die mit Alkoholen unter Carbonatbildung, bzw. mit Alkoholen und primären Aminen unter Urethanbildung reagieren, ausgewählt aus der Gruppe bestehend aus gegebenenfalls cyclischen Carbonaten der vorstehend genannten allgemeinen Formel (III), Harnstoff, Phosgen und Bischlorkohlensäureester von Alkoholen der vorstehend genannten allgemeinen Formel (I), wobei die als Komponente a) eingesetzten mehrwertigen Alkohole einerseits und die in Form der Bischlorkohlensäureester eingesetzten Alkohole der Formel (I) andererseits, jeweils der oben gemachten Definition von Q und n entsprechen, jedoch nicht identisch sein müssen. Bevorzugt werden beim erfindungsgemäßen Verfahren gegebenenfalls cyclische Carbonate der vorstehend genannten allgemeinen Formel (III) eingesetzt. Beispiele geeigneter Carbonate sind Dimethyl-, Diethyl-, Di-n-propyl-, Di-n-butyl-, Diphenyl-, Ethylen- und Propylencarbonat. Besonders bevorzugt wird Diphenylcarbonat verwendet.

Bei der Durchführung des erfindungsgemäßen Verfahrens werden die Reaktionspartner in den oben genannten Mengenverhältnissen eingesetzt.

Das erfindungsgemäße Verfahren kann ein- oder zweistufig durchgeführt werden.

Zur einstufigen Durchführung des erfindungsgemäßen Verfahrens kann beispielsweise so vorgegangen werden, daß die Polyhydroxylverbindungen a) und die Carbonate c), gegebenenfalls nach Aufschmelzen bei ca. 50 bis 80°C, homogenisiert werden, bevor die Aminkomponente b) unter Rühren zudosiert wird. Anschließend legt man Wasserstrahlvakuum an und erhöht die Temperatur auf ca. 110 bis 140°C, bis die Destillation der Hydroxylkomponente des eingesetzten Ausgangscarbonats beginnt. Entsprechend der Destillationsgeschwindigkeit wird die Innentemperatur allmählich bis auf schließlich maximal 200°C erhöht und solange dort belassen, bis die Destillation beendet ist. Letzte Spuren flüchtiger Bestandteile können durch Anlegen von Hochvakuum oder durch Azeotropdestillation unter Zudosierung von Schleppern wie z.B. Wasser oder Ethylenglykol entfernt werden.

Bei dieser einstufigen Verfahrensweise kommen pro eingesetztem Hydroxyläquivalent der Komponente a) und pro Mol Amin b) maximal 0,5 Mol Kohlensäurederivat, d.h. vorzugsweise Carbonat zum Einsatz. Da die Kondensationsreaktion bei Einsatz von äquivalenten Mengen Carbonat c) einerseits und Hydroxylkomponente a) und Aminkomponente b) andererseits gegen Ende der Reaktion immer langsamer verläuft, kann es vorteilhaft sein, einen Unterschuß an Carbonaten, bezogen auf die Menge an Hydroxylverbindungen und Aminkomponente, einzusetzen. Grundsätzlich ist jedoch darauf zu achten, daß die Mengenverhältnisse der Ausgangsmaterialien so gewählt werden, daß Umsetzungsprodukte mit den obengenannten Kenndaten bezüglich des Gehalts an Carbonatgruppen, Urethangruppen, Hydroxylgruppen und tertiären Aminogruppen resultieren.

Die Reaktion wird bevorzugt ohne Lösungsmittel durchgeführt, jedoch kann die Verwendung von Lösungsmitteln in einigen Fällen zweckmäßig bzw. erforderlich sein, z.B. bei Einsatz schwerlöslicher Ausgangsstoffe. In solchen Fällen kommen Lösungsmittel in Betracht, die nicht mit den Reaktionskomponenten reagieren und einen Siedepunkt aufweisen, der sich unter dem Siedepunkt der Hydroxylkomponente des eingesetzten Carbonats liegt, um eine einwandfreie destillative Trennung zu ermöglichen. Geeignet sind beispielsweise Kohlenwasserstoffe wie Toluol oder Xylol, halogenierte Kohlenwasserstoffe wie Chlorbenzol oder Dichlorbenzol, Ether wie Ethylenglykoldimethylether, Diethylenglykoldimethylether, Diphenylether oder Dioxan und Amide wie Dimethylformamid, Dimethylacetamid oder N-Methylpyrrolidon. In manchen Fällen kann der Zusatz von Katalysatoren wie Alkali-Basen oder Übergangsmetallverbindungen wie z.B. Dibutylzinnoxid, oder Tetrabutyltitanat vorteilhaft sein, jedoch ist eine Arbeitsweise ohne Katalysatorzusatz bevorzugt.

Bei der zweistufigen Durchführung des erfindungsgemäßen Verfahren wird zunächst in der ersten Stufe in an sich bekannter Weise aus der Polyolkomponente a) und dem Kohlensäurederivat c) nach bekannten Verfahren, vorzugsweise unter Einhaltung eines Molverhältnisses von Hydroxylgruppen der Komponente a) zu Kohlensäurederivaten c) von 2:1 bis 3:1, ein Polycarbonat hergestellt, welches anschließend im Sinne einer Aminolysereaktion der Carbonatgruppen mit Aminen b) zur Reaktion gebracht wird. Zu Polycarbonaten führende Umsetzungen von mehrwertigen Alkoholen mit Diarylcarbonaten sind z.B. in der DOS 2 555 805, mit Dialkylcarbonaten in der DOS 2 555 805, mit cyclischen Carbonaten (Dioxolanonen) in der DOS 1 915 908, mit Phosgen in der DOS 1 595 446, mit Bis-Chlorkohlensäureestern in DP 857 948 und mit Harnstoff in P. Ball, H. Fuillmann und W. Heitz in Angew. Chem. 92 1980 Nr. 9, S. 742, 743 beschrieben. Das Molekulargewicht Mn der Polycarbonate sollte möglichst hoch liegen, um genügend Carbonatgruppen für die Umsetzung mit der Aminkomponente b) zur Verfügung zu haben. Bevorzugt werden mittlere Molekulargewichte Mn von 1000 bis 4000. Die erhaltenen Polycarbonat-Zwischenprodukte werden anschließend mit Aminkomponenten b) umgesetzt. Dabei wird die Menge an Aminkomponente so bemessen, daß pro Mol Carbonatgruppe 0,2 bis 0,5 Mol Aminkomponente b) eingesetzt wird. Zur Durchführung der Reaktion kann man beispielsweise so vorgehen, daß man das gegebenenfalls aufgeschmolzene Polycarbonat bei Raumtemperatur oder bei erhöhter Temperatur von ca. 60 bis 80°C vorlegt und die Aminkomponente b) unter Rühren zugibt. Anschließend wird bei 80 bis 120°C noch solange nachgerührt, bis die primären Aminogruppen der Aminkomponente abreagiert sind. Dies kann z.B. durch Titration mit HCl oder Perchlorsäure festgestellt werden.

Die 2. Stufe dieser Variante des erfindungsgemäßen Verfahrens wird bevorzugt ohne Lösungsmittel durchgeführt, jedoch kann die Verwendung von Lösungsmitteln in einigen Fällen zweckmäßig bzw, erforderlich sein, z.B. bei Einsatz schwerlöslicher Ausgangsstoffe. In solchen Fällen kommen Lösungsmittel in Betracht, die nicht mit den Reaktionskomponenten reagieren. Geeignet sind z.B. die oben aufgeführten Lösungsmittel.

Auch bei der zweistufigen Durchführung des erfindungsgemäßen Verfahrens werden Art und Mengenverhältnisse der Ausgangsmaterialien im Rahmen der gemachten Offenbarung so gewählt, daß letztendlich Verfahrensprodukte mit den vorstehend genannten Kenndaten bezüglich des Gehalts an Carbonatgruppen, Urethangruppen, tert. Aminogruppen und Hydroxylgruppen resultieren.

Die erfindungsgemäßen, tertiäre Aminogruppen aufweisenden Verbindungen sind weitgehend geruchsfrei. Durch geeignete Wahl der Ausgangsmaterialien, insbesondere der Hydroxylverbindungen a) werden Produkte erhalten, die eine ausgezeichnete Verträglichkeit mit den in der Polyurethanchemie üblichen Polyetherpolyolen aufweisen. Sie können daher vorteilhaft als Katalysatoren bzw. Aktivatoren zur Herstellung von Polyurethanen, insbesondere von Polyurethanschaumstoffen aus den üblichen Ausgangsmaterialien Anwendung finden.

Bei dieser erfindungsgemäßen Verwendung werden die erfindungsgemäßen Verbindungen im allgemeinen in Mengen von 1 bis 20 Gew.-%, bezogen auf das gesamte, zu Polyurethanschaumstoff ausreagierende Reaktionsgemisch eingesetzt. Im allgemeinen handelt es sich bei den erfindungsgemäßen Verbindungen lediglich um Hilfsmittel, die neben den üblichen Polyhydroxylverbindungen, insbesondere Polyetherpolyolen, und gegebenenfalls neben mitverwendetem Wasser als Reaktionspartner für die organischen Polyisocyanate zum Einsatz gelangen. Vorzugsweise wird die Menge der erfindungsgemäßen Verbindungen bei der Herstellung von Polyurethanschaumstoffen so gewählt, daß in dem Reaktionsgemisch 0,01 bis 1 Gew.-% an tertiären Stickstoffatomen der erfindungsgemäßen Verbindungen vorliegen. Die erfindungsgemäßen Verbindungen eignen sich besonders bevorzugt als Katalysatoren bei der Herstellung von Verbundkörpern durch Hinterschäumen von Kunststoffolien mit einem zu Polyurethanschaumstoff ausreagierenden Reaktionsgemisch.

Neben der erfindungsgemäßen Verwendung können die erfindungsgemäßen Verbindungen auch noch für andere Einsatzgebiete Verwendung finden. Beispielhaft genannt seien die Verwendung als Oberflächen-aktive Mittel (auch in Form ihrer Ammoniumsalze) Emulgatoren oder als Stabilisatoren.

In den nachfolgenden Beispielen beziehen sich alle Prozentangaben auf das Gewicht.

### Beispiele

### Herstellung von Ausgangsmaterialien:

### Polycarbonat A:

3686 g (19,2 mol) Tripropylenglykol, 858 g (6,4 mol) Trimethylolpropan, 4794 g (22,4 mol) Diphenylcarbonat und 2 g Dibutylzinnoxid werden bei 12 mbar allmählich auf 180°C erhitzt, wobei Phenol abdestilliert. Wenn bei 180°C/12 mbar nichts mehr abdestilliert, wird zur Entfernung von restlichem Phenol noch 2 Stunden bei 160°C an der Ölpumpe bei 0,5 mbar nachgerührt.
OH-Zahl: 136
Molekulargewicht Mn: 1650 g
Viskosität/25°C: 55.740 mPa.s

### Polycarbonat B:

2894 g (21,6 mol) Dipropylenglykol, 965 g (7,2 mol) Trimethylolpropan, 5393 g (25,2 mol) Diphenylcarbonat und 5 g Dibutylzinnoxid werden analog Polycarbonat A umgesetzt.
OH-Zahl: 162
Molekulargewicht Mn: 1385 g
Viskosität/25°C: 117.920 mPa.s

### Polycarbonat C:

3725 g (19,2 mol) Triethylenglykol, 858 g (6,4 mol) Trimethylolpropan, 4794 g (22,4 mol) Diphenylcarbonat und 2 g Dibutylzinnoxid werden analog Polycarbonat A umgesetzt.
OH-Zahl: 129,2
Molekulargewicht Mn: 1737
Viskosität/25°C: 22.560 mPa.s

### Beispiel 1 (einstufige Reaktion)

1188 g (7,92 mol) Triethylenglykol und 2080 g (9,72 mol) Diphenylcarbonat werden bei 80°C unter Stickstoff aufgeschmolzen. Hierzu tropft man unter Rühren 367 g (3,6 mol) 1-(Dimethylamino)-3-aminopropan über ca. 1 Stunde zu, legt dann Wasserstrahlvakuum an und erhöht die Innentemperatur auf 160°C, wobei Phenol abdestilliert. Wenn bei 160°C/12 mbar nicht mehr abdestilliert, wird zur Entfernung von restlichem Phenol noch 2 Stunden bei 160°C an der Ölpumpe bei 0,5 mbar nachgerührt.
Viskosität/25°C: 1.960 mPa.s
Carbonatgruppen: 22,6 %
Urethangruppen: 13,1 %
Hydroxylgruppen: 0 %
tert. Stickstoff: 2,8 %

### Beispiel 2 (einstufige Reaktion)

1286 g (6,70 mol) Tripropylenglykol, 299 g (2,23 mol) Trimethylolpropan, 1673 g (7,82 mol) Diphenylcarbonat und 341 g (3,35 mol) 1-(Dimethylamino)-3-aminopropan werden analog Beispiel 1 umgesetzt.
Viskosität/25°C: 4.430 mPa.s
Carbonatgruppen: 12,6 %
Urethangruppen: 9,28 %
Hydroxylgruppen: 6,25 %
tert. Stickstoff: 2,2 %

### Beispiel 3 (zweistufige Reaktion)

Zu 1400 g Polycarbonat A tropft man bei 50°C in Stickstoffatmosphäre unter gutem Rühren 267 g (2,62 mol) 1-(Dimethylamino)-3-aminopropan zügig zu und rührt erst 2 Stunden bei 100°C, dann noch 5 Stunden bei 120°C.
Viskosität/25°C: 4.320 mPa.s
Carbonatgruppen: .12,6 %
Urethangruppen: 9,27 %
Hydroxylgruppen: 6,24 %
tert. Stickstoff: 2,2 %

### Beispiel 4 (zweistufige Reaktion)

640 g Polycarbonat B und 286 g (2,62 mol) 1-(Dimethylamino)-3-aminopropan werden analog Beispiel 3 umgesetzt.
Viskosität/25°C: 4.100 mPa.s
Carbonatgruppen: 6,16 %
Urethangruppen: 16,7 %
Hydroxylgruppen: 8,48 %
tert. Stickstoff: 3,96 %

### Beispiel 5 (zweistufige Reaktion)

700 g Polycarbonat B und 243 g (1,674 mol) N,N-bis-(3-Aminopropyl)-methylamin werden analog Beispiel 3 umgesetzt.
Viskosität/25°C: 1.140 mPa.s
Carbonatgruppen: 14,2 %
Urethangruppen: 10.5 %
Hydroxylgruppen: 7,04 %
tert. Stickstoff: 2,49 %

### Beispiel 6 (zweistufige Reaktion)

1400 g Polycarbonat B und 456 g (4,47 mol) 1-(Dimethylamino)-3-aminopropan werden analog Beispiel 3 umgesetzt.
Viskosität/25°C: 6.320 mPa.s
Carbonatgruppen: 10,83 %
Urethangruppen: 14,2 %
Hydroxylgruppen: 8,18 %
tert. Stickstoff: 3,37 %

### Beispiel 7 (zweistufige Reaktion)

1400 g Polycarbonat C und 354 g (3,47 mol) 1-(Dimethylamino)-3-aminopropan werden analog Beispiel 3 umgesetzt.
Viskosität/75°C: 60 mPa.s
Carbonatgruppen: 8,89 %
Urethangruppen: 11,67 %
Hydroxylgruppen: 6,73 %
tert. Stickstoff: 2,77 %

### Anwendungsbeispiele

### Polyolformulierung

48 Gew.-Teile eines Polyetherpolyols der OH-Zahl 28, hergestellt durch Propoxylierung von Trimethylolpropan und anschließender Ethoxylierung des Propoxylierungsproduktes (PO:EO-Gewichtsverhältnis = 82,5:17,5) sowie 44 Gew.-Teile eines analog auf Propylenglykol aufgebauten Polyetherpolyols der OH-Zahl 28 werden in Parallelversuchen mit der in Tabelle 1 angegebenen Menge an Aktivator vermischt. Als Treibmittel werden 2,5 Gew.-Teile Wasser zugegeben.

### Polyisocyanatkomponente

In den nachfolgenden Beispielen wurde jeweils ein Polyisocyanatgemisch der Diphenylmethanreihe (Gemische aus Diisocyanatophenylmethan-Isomeren und deren höheren Homologen) der Viskosität (23°C) 200 mPa.s mit einem NCO-Gehalt von 32 Gew.-% verwendet.

### Herstellung von Schaumstoffen

Die Herstellung der Schaumstoffe erfolgte jeweils nach der Methode der Handverschäumung. Dabei werden alle Komponenten mit Ausnahme der Polyisocyanatkomponente während 30 s vorgerührt (Rührgeschwindigkeit: 1000 U/Min). Anschließend wird die Isocyanatkomponente zugesetzt und während 10 s bei Raumtemperatur weiter gerührt. Das Mischungsverhältnis entspricht dabei einer Isocyanatkennzahl von 100.

Die Reaktivität der Polyolkomponente wurde durch die Start-, Steig-, Fließ- und Abbindezeit in Parallelversuchen bestimmt, wobei die Polyolformulierung wie beschrieben mit der Polyisocyanatkomponente in einem Becherglas unter Rühren bei Raumtemperatur vereinigt wird. Die Startzeit ist die Zeit, die vom Zeitpunkt der Polyisocyanatzugabe bis zum Beginn des Schäumvorganges verstreicht; die Steigzeit ist die Zeit, die vom Zeitpunkt der Polyisocyanatzugabe bis zum Ende des Schäumvorganges verstreicht; die Abbindezeit ist die Zeit, die vom Zeitpunkt der Polyisocyanatzugabe bis zur Klebfreiheit des Schaumstoffes verstreicht. Die Fließzeit errechnet sich aus der Differenz von Steig- und Startzeit. Das Raumgewicht ist die Dichte, die sich nach Abschneiden der Schaumkuppe in einem 660 ml Becher errechnet.

| Aktivator | Startzeit (s) | Steigzeit (s) | Fließzeit (s) | Abbindezeit (s) | Raumgewicht (g/l) | Gew.-Teile |
|---|---|---|---|---|---|---|
| aus Bsp. 1 | 14 | 150 | 136 | 172 | 58 | 3,0 |
| aus Bsp. 3 | 15 | 125 | 115 | - | 56 | 3,0 |
| aus Bsp. 6 | 13 | 85 | 72 | 95 | 53 | 3,0 |
| aus Bsp. 7 | 14 | 92 | 78 | 105 | 55 | 3,0 |

## Patentansprüche

1. Verfahren zur Herstellung von Carbonat- und Urethangruppen, sowie gegebenenfalls Hydroxylgruppen aufweisenden tert.-Aminen, dadurch gekennzeichnet, daß man
a) eine Polyolkomponente, bestehend aus mindestens einem mehrwertigen Alkohol der Formel
Q(OH)ₙ (I)
und
b) eine Aminkomponente, bestehend aus mindestens einem primärtertiären Diamin der Formel 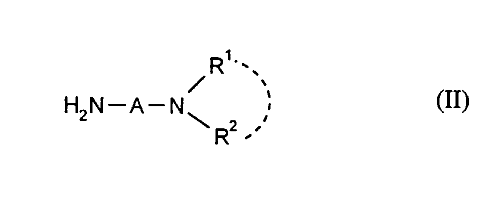 mit
c) Kohlensäurederivaten, ausgewählt aus der Gruppe bestehend aus Carbonaten der Formel 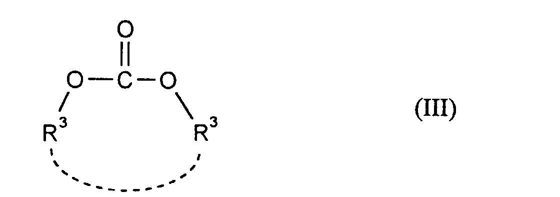 Harnstoff, Phosgen und Bischlorkohlensäureester von Polyolen der Formel (I),
ein- oder zweistufig unter Carbonat- und Urethanbildung zur Reaktion bringt,
mit der Maßgabe, daß das Mengenverhältnis der Komponenten a) und b) einem Molverhältnis von Hydroxylgruppen zu primären Aminogruppen von 4:1 bis 16:1 entspricht und die Kohlensäurederivate c) in solchen Mengen zum Einsatz gelangen, daß auf jedes Mol an Aminen b) 0,5 Mol an Kohlensäurederivat c) und zusätzlich auf jedes Mol an Hydroxylgruppen der Komponente a) 0,2 bis 0,5 weitere Mole an Kohlensäurederivaten c) entfallen, wobei
Q für den Rest steht, wie er durch Entfernung von n-Hydroxylgruppen aus einem n-wertigen, gegebenenfalls Ethergruppen aufweisenden aliphatischen Alkohol des Molekulargewichtsbereichs 90 bis 400 erhalten wird,
n für 2 oder 3 steht,
A für einen zweiwertigen aliphatischen Kohlenwasserstoffrest mit 2 bis 6 Kohlenstoffatomen steht, wobei zwischen den Stickstoffatomen mindestens 2 Kohlenstoffatome angeordnet sind,
R¹ und R² für gleiche oder verschiedene Reste stehen und Alkylreste mit 1 bis 4 Kohlenstoffatomen bedeuten, die auch zusammen mit dem Stickstoffatom und gegebenenfalls weiteren Heteroatomen einen gesättigten, heterocyclischen, gegenüber den Kohlesäurederivaten c) inerten Ring mit 5 oder 6 Ringgliedern bilden können, und
R³ jeweils für Phenylreste oder Alkylreste mit 1 bis 4 Kohlenstoffatomen stehen oder zusammen einen Alkylenrest mit 2 oder 3 Kohlenstoffatomen bilden.

2. Verfahren gemäß Anspruch 1, dadurch gekennzeichnet, daß man in einer ersten Reaktionsstufe aus einer Alkoholkomponente a) und einem Kohlensäurederivat c) in an sich bekannter Weise unter Einhaltung eines Molverhältnisses von Hydroxylgruppen der Komponente a) zu Kohlensäurederivaten c) von 2:1 bis 3:1 ein Hydroxylgruppen aufweisendes Polycarbonat herstellt und dieses anschließend in einer zweiten Reaktionsstufe im Sinne einer Aminolysereaktion unter Einhaltung eines Molverhältnisses von primären Aminogruppen zu Carbonatgruppen von 0,2:1 bis 0,5:1 mit Aminen b) unter Urethanbildung umsetzt.

3. Gemäß Anspruch 1 und 2 erhältliche, Carbonat- und Urethangruppen, sowie gegebenenfalls Hydroxylgruppen aufweisende tert. Amine.

4. Verwendung der gemäß Anspruch 1 und 2 erhältlichen tert. Amine als gegebenenfalls einbaufähige Katalysatoren für die Isocyanat-Additionsreaktion bei der Herstellung von Polyurethankunststoffen nach dem Isocyanat-Polyadditionsverfahren.

5. Verwendung gemäß Anspruch 4 bei der Herstellung von Verbundkörpern aus Kunststoffolien und Polyurethanschaumstoff durch Hinterschäumen von Kunststoffolien mit einem zu Polyurethanschaumstoff ausreagierenden Reaktionsgemisch.

## Claims

1. Process for the production of tert. amines exhibiting carbonate and urethane groups, and optionally hydroxyl groups, characterised in that
a) a polyol component consisting of at least one polyhydric alcohol of the formula
Q(OH)ₙ (I)
and
b) an amine component consisting of at least one primary-tertiary diamine of the formula 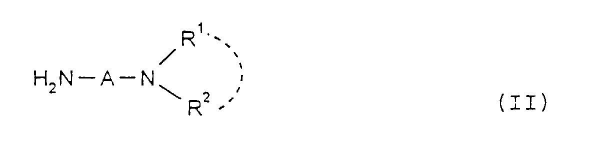 are reacted with
c) carbonic acid derivatives selected from the group consisting of carbonates of the formula 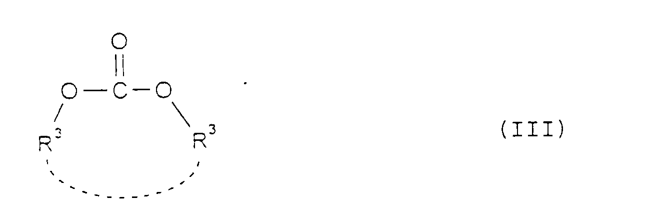 urea, phosgene and bischlorocarbonates of polyols of formula (I),
in one or two steps with the formation of carbonates and urethanes,
on the condition that the quantity ratio of components a) and b) corresponds to a molar ratio of hydroxyl groups to primary amino groups of 4:1 to 16:1 and the carbonic acid derivatives c) are used in quantities such that for each mole of amines b) there are 0.5 moles of carbonic acid derivative c) and additionally for each mole of hydroxyl groups of component a) there are a further 0.2 to 0.5 moles of carbonic acid derivatives c), wherein
Q denotes the residue which is obtained by the removal of n-hydroxyl groups from an n-hydric aliphatic alcohol, optionally exhibiting ether groups, in a molecular weight range of 90 to 400,
n denotes 2 or 3,
A denotes a divalent, aliphatic hydrocarbon residue with 2 to 6 carbon atoms, wherein at least 2 carbon atoms are arranged between the nitrogen atoms,
R¹ and R² denote the same or different residues and mean alkyl residues with 1 to 4 carbon atoms which, together with the nitrogen atom and optionally further heteroatoms, may also form a saturated, heterocyclic ring with 5 or 6 ring links which is inert with respect to the carbonic acid derivatives c), and
R³s each denote phenyl residues or alkyl residues with 1 to 4 carbon atoms or together form an alkylene residue with 2 or 3 carbon atoms.

2. Process according to claim 1, characterised in that, in a first reaction step, a polycarbonate exhibiting hydroxyl groups is produced from an alcohol component a) and a carbonic acid derivative c) by a method which is known per se, maintaining a molar ratio of hydroxyl groups of component a) to carbonic acid derivatives c) of 2:1 to 3:1, and this is subsequently reacted in a second reaction step with amines b), maintaining a molar ratio of primary amino groups to carbonate groups of 0.2:1 to 0.5:1, in the sense of an aminolysis reaction, with the formation of urethanes.

3. Tert. amines exhibiting carbonate and urethane groups, and optionally hydroxyl groups, obtainable according to claims 1 and 2.

4. Use of the tert. amines obtainable according to claims 1 and 2 as catalysts, which are optionally capable of incorporation, for the isocyanate addition reaction in the production of polyurethane plastics by the isocyanate polyaddition process.

5. Use according to claim 4 in the production of composites of plastics films and polyurethane foam by the foam-backing of plastics films with a reaction mixture reacting to give polyurethane foam.

## Revendications

1. Procédé de préparation d'amines tertiaires contenant des groupes carbonate et uréthane et le cas échéant des groupes hydroxy, caractérisé en ce que l'on fait réagir en un ou deux stades opératoires avec formation de carbonates et d'uréthanes
a) un composant polyol consistant en au moins un alcool polyvalent de formule
Q(OH)ₙ (I)
et
b) un composant amine consistant en au moins une diamine primaire-tertiaire de formule 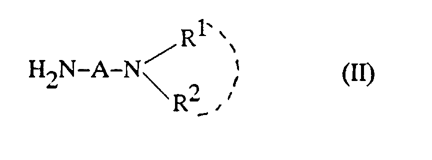 avec
c) des dérivés de l'acide carbonique choisis dans le groupe consistant en les carbonates de formule 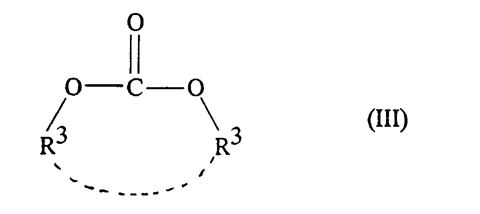 l'urée, le phosgène et les esters bis-chlorocarboniques des polyols de formule (I),
sous réserve que les proportions relatives des composants a) et b) correspondent à un rapport molaire de 4 : 1 à 16 : 1 entre les groupes hydroxy et les groupes amino primaires et que les dérivés de l'acide carbonique c) sont mis en oeuvre en quantité telle que, pour chaque mole d'amine b), on dispose de 0,5 mol du dérivé d'acide carbonique c) et qu'en outre, pour chaque mole, de groupes hydroxy du composant a), on dispose de 0,2 à 0,5 mol en plus des dérivés de l'acide carbonique c), les symboles des formules ci-dessus ayant les significations suivantes :
Q représente le radical obtenu par élimination de n groupes hydroxy d'un alcool aliphatique de valence n contenant le cas échéant des groupes éthers, à un poids moléculaire dans l'intervalle de 90 à 400,
n est égal à 2 ou 3,
A représente un radical hydrocarboné aliphatique divalent en C₂-C₆, les atomes d'azote étant séparés par au moins deux atomes de carbone,
R¹ et R², ayant des significations identiques ou différentes, représentent chacun un groupe alkyle en C₁-C₄ ou forment ensemble et avec l'atome d'azote et le cas échéant d'autres hétéroatomes un hétérocycle saturé à 5 ou 6 chaînons inerte à l'égard des dérivés de l'acide carbonique c), et
chacun des symboles R³ représente un groupe phényle ou alkyle en C₁-C₄ ou bien les deux symboles R³ représentent ensemble un groupe alkylène en C₂ ou C₃.

2. Procédé selon revendication 1, caractérisé en ce que, dans un premier stade de réaction, on prépare à partir d'un composant alcool a) et d'un dérivé de l'acide carbonique c), de manière connue en soi, en maintenant un rapport de 2 : 1 à 3 : 1 entre les groupes hydroxy du composant a) et les dérivés de l'acide carbonique c), un polycarbonate contenant des groupes hydroxy qu'on fait ensuite réagir dans un deuxième stade de réaction, dans une réaction d'aminolyse, en maintenant un rapport molaire de 0,2 : 1 à 0,5 : 1 entre les groupes amino primaires et les groupes carbonate, avec les amines b), avec formation d'uréthanes.

3. Amines tertiaires contenant des groupes carbonate et uréthane et le cas échéant des groupes hydroxy, obtenues selon les revendications 1 et 2.

4. Utilisation des amines tertiaires obtenues selon les revendications 1 et 2 en tant que catalyseurs, éventuellement combinables chimiquement, pour la réaction d'addition des isocyanates, à la préparation de résines synthétiques de polyuréthanes par le procédé de polyaddition des isocyanates.

5. Utilisation selon revendication 4 pour la fabrication de corps composites consistant en feuilles de résines synthétiques et mousse de polyuréthane par gonflement en mousse, à l'arrière de feuilles de résines synthétiques, d'un mélange de réaction capable de réagir avec formation d'une mousse de polyuréthane.
